# EUROPEAN PATENT APPLICATION

(11) **EP 4 725 525 A1**
(43) Date of publication of application: **15.04.2026**
(21) Application number: 24306647.9
(22) Date of filing: 08.10.2024
(51) Int. Cl.: A61M 5/20, A61M 5/24, A61M 5/315, A61M 5/32

(54) **SAFETY SYSTEM FOR AN INJECTION DEVICE AND INJECTION DEVICE COMPRISING SUCH A SAFETY SYSTEM**

(71) Applicant: NEMERA LA VERPILLIERE, 38290 La Verpillière (FR)
(72) Inventor: DELVALAC, Sébastien, 69007 Lyon (FR)
(74) Representative: Lavoix

(57) **Abstract**

This safety system (12) for an injection device (2) comprises a sleeve (14) fixed in a body (6) of the injection device, a needle cover (18) able to translate with respect to the sleeve (14) between a first position, a second position and a third second position in two opposite longitudinal directions (D2, D2') and a locker (20) able to translate in a longitudinal direction (D2) and configured to be selectively coupled to the needle cover (18) and to the sleeve (14). The needle cover (18) is configured to translate, in a first longitudinal direction (D2), from the first position to the second position, along with the locker (20). The needle cover (18) is configured to translate with respect to the locker (20), in a second longitudinal direction (D2'), opposite to the first longitudinal direction, from the second position to the third position. The third position is the same as the first position. After moving from the second position to the third position, the needle cover (18) is locked in the third position.

## Description

The present invention concerns a safety system for an injection device. The present invention also concerns an injection device comprising, amongst others, a safety system for shielding a needle from the exterior and preventing needlestick injuries, in particular after making the injection.

### TECHNICAL FIELD OF INVENTION

The invention belongs to the technical field of hiding a needle used in an injection device during use and preventing needle exposure after use. This protects the users of an injection device against injuries due to a needle and it helps the people with needlephobia to use the injection device. In this technical field, it is known to use a needle cover to partially isolate a needle from the exterior of an injection device and to lock the needle cover after use.

With this respect, EP3138598B1 discloses a safety shield system, where a shield moves axially during the use of a pen injector. Following injection, some fixedly arranged hook shaped fingers lock the shield in an extended position, different from a starting position. Locking of the shield in the extended position takes place by direct cooperation between the shield and the fingers.

On the other hand, EP1949929B1 discloses a passive safety shield system, where an injection end shield provides protection by locking itself, in a safety position different from a starting position, against a hub via an interlocked sleeve. Several radial snaps of the shield engage into corresponding cut-outs of the sleeve when the shield is in the safety position. Holding of the shield in the safety position is not guaranteed.

These known systems are complicated and may be subject to malfunctions.

A technical problem is how to provide a reliable safety system to hide a needle of an injection device and lock a needle cover in a safety position, after use of the injection device, in order to prevent needle-stick injuries.

### SUMMARY OF THE INVENTION

The present invention addresses this issue by providing a safety system for an injection device, capable of efficiently holding a needle cover in a safety position, this system being reliable and easy to manufacture and assemble.

With this respect, according to a first aspect, the present invention relates to a safety system for an injection device comprising a body, the safety system comprising
- a sleeve fixed in the body;
- a needle cover able to translate with respect to the sleeve between a first position, a second position and a third second position in two opposite longitudinal directions;
- a locker able to translate in a longitudinal direction and configured to be selectively coupled to the needle cover and to the sleeve,
wherein
- the needle cover is configured to translate, in a first longitudinal direction, from the first position to the second position, along with the locker;
- the needle cover is configured to translate with respect to the locker, in a second longitudinal direction, opposite to the first longitudinal direction, from the second position to the third position;
- the third position is the same as the first position; and
- after moving from the second position to the third position, the needle cover is locked in the third position.

Thanks to the invention, the needle cover is subjected to an axial back-and-forth movement between only two positions, without any rotation of the needle cover. Before use of the injection device, the needle cover is extended in a first position, where it shields the needle. Upon use of the injection device, the needle cover retracts toward a second position where it unveils the needle. After the injection, the needle cover moves to the third position in sequence, which is the same as the first position, where it shields the needle. Moreover, due to the fact that the locker moves together with the needle cover, between its first and second positions, the locker can be brought to a position where it locks with respect to the sleeve when the needle cover is in its second position. This allows the locker to efficiently keep the needle cover in the third position, when needed.

According to advantageous and optional aspects of the invention, such a safety system may incorporate one or several of the following features:
- The sleeve and the body are two separate pieces, fixedly assembled together.
- The sleeve is in one piece with the body.
- The locker is ring shaped and mounted around the needle cover in assembled configuration of the safety system.
- When it reaches the second position, the locker couples with the sleeve and becomes fixed with the sleeve.
- The safety system further comprises a biasing element for biasing the needle cover toward its first position.
- The needle cover comprises at least one longitudinal slot; the sleeve comprises at least one key element protruding radially with respect to a peripheral surface of the sleeve; each key element is engaged in one longitudinal slot of the needle cover and travels along this longitudinal slot when the needle cover moves from the first position to the second position and from the second position to the third position.
- The needle cover includes two elongated portions disposed around the sleeve; each elongated portion is equipped with a longitudinal slot; the sleeve comprises two key elements, preferably diametrically opposed; and each key element protrudes outwardly from the outer peripheral surface and is engaged in a respective longitudinal slot.
- The locker includes at least one aperture; the sleeve comprises at least one key element protruding radially with respect to a peripheral surface of the sleeve; each key element engages into one aperture of the locker when the needle cover reaches its second position and remains in this aperture when the needle cover moves from the second position to the third position.
- Engagement of the key element into the aperture occurs by elastic deformation of the locker.
- The key element engaged in the longitudinal slot is the same as the key element, which engages into the aperture when the needle cover reaches its second position.
- The locker comprises at least one locking heel engaged in a corresponding recess of the needle cover, when the needle cover is in its first position before moving to its second position and when the needle cover moves from its first position to its second position, and wherein the heel enters the longitudinal slot of the needle cover when the needle cover reaches its second position and stays in the longitudinal slot when the needle cover moves from its second position to its third position.
- The heel is provided on a deformable tab of the locker and extends radially with respect to this deformable tab.
- The biasing element has a form of a helical spring disposed between the sleeve and the needle cover.

According to a second aspect, the invention relates to an injection device comprising a syringe, with at least a barrel and a needle, and a safety system as mentioned here above for shielding the needle from the exterior.

According to advantageous and optional aspects of the invention, such a safety system may incorporate one or several of the following features:
- The injection device comprises a reusable module and a disposable module, which includes the safety system.
- The reusable module comprises a frame and a pushing element, wherein after connecting the reusable module with the disposable module, the pushing element is coupled with the needle cover in translation with reference to the frame.
- The biasing element is part of the reusable module and located between the pushing element and the frame.
- The injection device comprises a motor for providing a force for the injection.

### BRIEF DESCRIPTION OF THE DRAWINGS

The invention will be better understood, based on the following description, given as a non-limiting example and made in reference to the following figures:
- [Fig. 1] Figure 1 is a perspective view of a first embodiment of an injection device according to a first embodiment of the invention, which includes a safety system according to a first embodiment of the invention;
- [Fig.2] Figure 2 is a perspective exploded view of the injection device of figure 1, together with a syringe used with this injection device;
- [Fig.3] Figure 3 represents, on two inserts A) and B) the safety system and the syringe, in a first position of a needle cover;
- [Fig. 4] Figure 4 represents, on two inserts A) and B) the safety system and the syringe, in a second position of the needle cover;
- [Fig. 5] Figure 5 represents on two inserts A) and B) the safety system and the syringe, in a third position of the needle cover;
- [Fig. 6] Figure 6 shows, on three inserts A), B) and C), a sleeve, a needle cover and a locker of the safety system, seen from different angles and at different scales;
- [Fig. 7] Figure 7 is a front view of an injection device according to a second embodiment of the invention, comprising a safety system according to a second embodiment the invention; and
- [Fig. 8] Figure 8 is a perspective exploded view of the injection device of figure 7, without a syringe.

### DETAILED DESCRIPTION OF SOME EMBODIMENTS

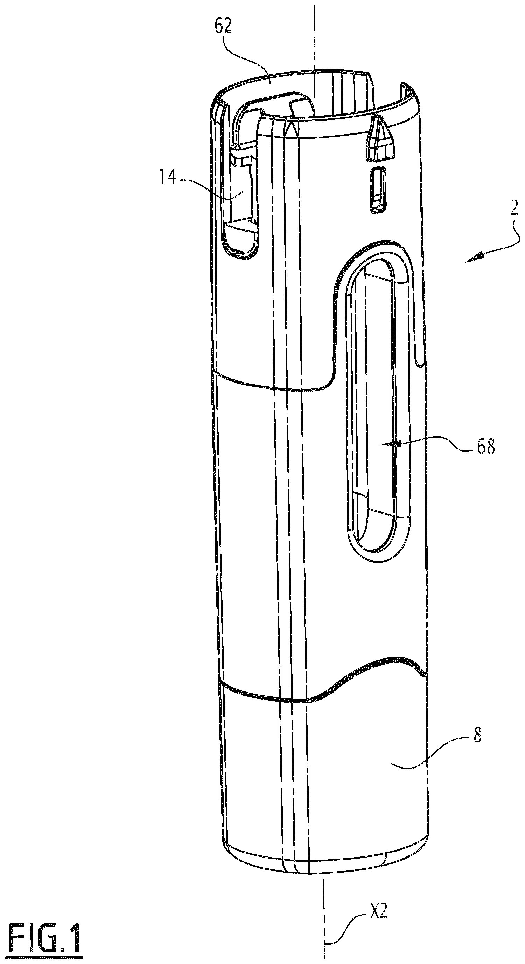

A first embodiment of an injection device 2 according to the invention is represented on figures 1 to 6 and is configured to accommodate a syringe 4, which includes a barrel 42, a needle 44, a piston 46 and a needle cap 48.

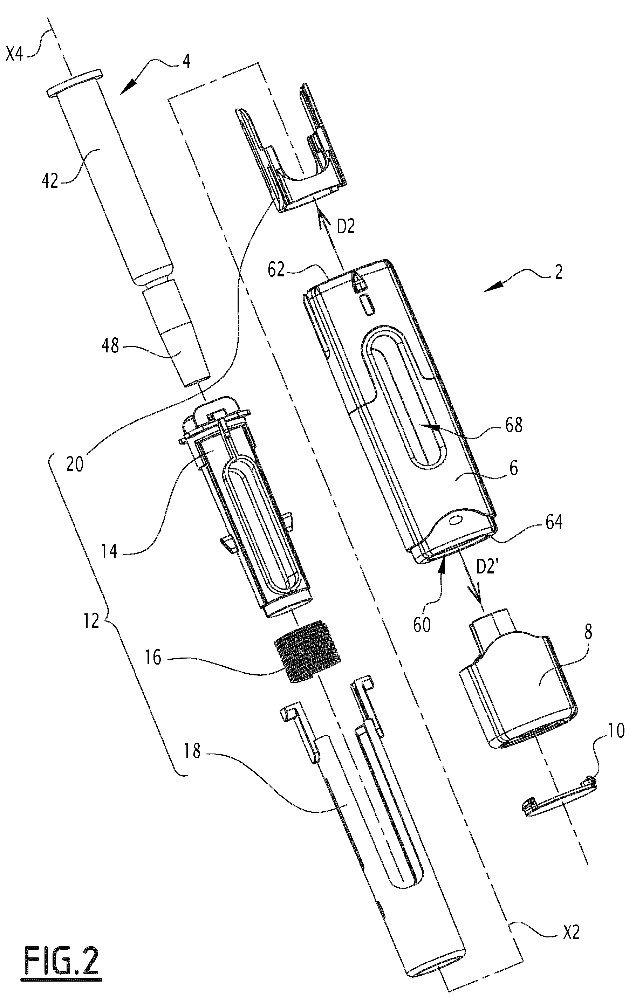

The syringe 4, which belongs to the injection device 2 when it is mounted within the injection device 2, is visible on figures 2 to 5. Advantageously, the barrel 42 is transparent.

The injection device 2 includes a body 6, a puller 8 and a cap 10, which closes the puller 8 on its side opposite to the body 6. The puller 8 allows extracting the needle cap 48 from the body 6, for example by providing friction interface between these elements. when it is necessary to use the needle 44 for injecting a liquid contained in the barrel 42, in particular into a human or animal body.

The injection device 2 extends along a longitudinal axis X2. The syringe 4 extends along a longitudinal axis X4. When the syringe 4 is mounted within the injection device 2, the axes X2 and X4 coincide.

62 denotes a first longitudinal end of the body 6, which preferably defines an entry zone for the syringe 4 when the syringe is inserted into the injection device 2.

64 denotes a second longitudinal end of the body 6, which is opposite to the first longitudinal end 62 and close to the puller 8 when the puller is mounted on the body 6.

D2 denotes a first longitudinal direction, parallel to the longitudinal axis X2 and oriented from the second end 64 to the first end 62. D2' denotes a second longitudinal direction, opposite to the first longitudinal direction, parallel to the longitudinal axis X2 and oriented from the first end 62 to the second end 64.

The second longitudinal end 64 defines an end opening 60 of the body 6, through which the needle 44 protrudes out of the injection device 2 when the liquid contained in the barrel 42 is to be injected within a human or animal body.

The injection device 2 is equipped with a safety system 12 for preventing needle-stick injuries when the injection device is manipulated, preferably by a patient or a medical practitioner.

The safety system 12 includes a sleeve 14 fixedly assembled within the body 6. In other words, the sleeve 14 is fixed in the body 6, along the longitudinal axis X2 and in any direction perpendicular to this axis.

The fixation of the sleeve 14 within the body 6 may occur in any conventional matter, e.g. by snapping, gluing, welding.

In a non-represented alternative embodiment of the invention, the sleeve 14 may be formed in one piece with the body 6. In other words, the body 6 and the sleeve 14 are monobloc. Such a case should also be regarded as when the sleeve 14 fixed with the body 6, and it is to be understood that the sleeve 14 fixed with the body covers also said monobloc possibility.

The sleeve 14 can be made, for example of, a polymer suitable for injection molding which is preferred way of manufacturing such element. Suitable polymer materials may be composed mainly of mainly of a copolymer of methyl methacrylate, acrylonitrile, butadiene and styrene (MABS), a copolymer of methyl methacrylate butadiene styrene (MMBS), polypropylene (PP), copolymer of acrylonitrile-butadiene-styrene (ABS), polyoxymethylene (POM), polyamide (PA), polybuthylene terephtalate (PBT), or a polycarbonate (PC) or their mixtures.

The sleeve 14 includes a body 142, which extends along a longitudinal axis X14. The body 142 is provided with two key elements 144 extending outwardly with respect to an external peripheral surface S142 of the body 142 and diametrically opposed with respect to the longitudinal axis X14.

In a non-represented variant of the invention, the sleeve 14 includes one key element 144 only.

The sleeve 14 also includes two external ribs 146 which surround two through-openings 148. These openings 148 are aligned with corresponding openings 68 formed in the body 6 for the inspection of the content of the barrel 42 when the syringe 4 is mounted within the injection device 2. The fact that the barrel is advantageously transparent helps the visualization of its content.

The safety system 2 also includes a biasing element 16.

In a non-limiting example, this biasing element 16 is a spiral, compression spring as shown on the figures. The spring is preferably made of a metal.

In a variant, this biasing element 16 can be made of a leaf spring, a conical spring, a flexible part of the body 6 or the sleeve 14 or any other means providing biasing force along the axis X2.

The safety system 12 also includes a needle cover 18 configured for surrounding the needle 44, in particular when the needle cap 48 has been removed, in order to hide the needle and prevent needle-stick injuries.

The needle cover 18 is preferably also made of a polymer which can be the same as, or different from, the material of the sleeve 14.

The needle cover 18 includes a substantially tubular body 182 centered on a longitudinal axis X18 and equipped with two longitudinal slots 184, which are parallel to the longitudinal axis X18 and which are located diametrically oppositely with respect to this axis. These longitudinal slots 184 are advantageously identical.

The longitudinal slots 184 are made through two elongated portions 185 of the body 182.

The needle cover 18 is also provided with two recesses 186, located diametrically oppositely with respect to its longitudinal axis X18. Advantageously, the recesses are realized in the form of through-holes, radially crossing the tubular body 182. An edge of each recess 186, which delimits this recess on the side of the adjacent longitudinal slot 184, is inclined with respect to the longitudinal axis X18 and forms a ramp surface S186, which diverges from this axis in the direction of the adjacent longitudinal slot 184.

The needle cover 18 is also equipped with two lugs 188, located diametrically oppositely with respect to its longitudinal axis X18, and two notches 189 opened towards the lugs 188, also located diametrically oppositely with respect to its longitudinal axis X18, but in the transvers direction perpendicular to the transverse direction along which the lugs are located. The notches are defined between the two elongated portions 185 of the body 182.

In a non-represented variant of the invention, the needle cover 18 includes one recess 186, one elongated portion185 and/or one lug 188 only.

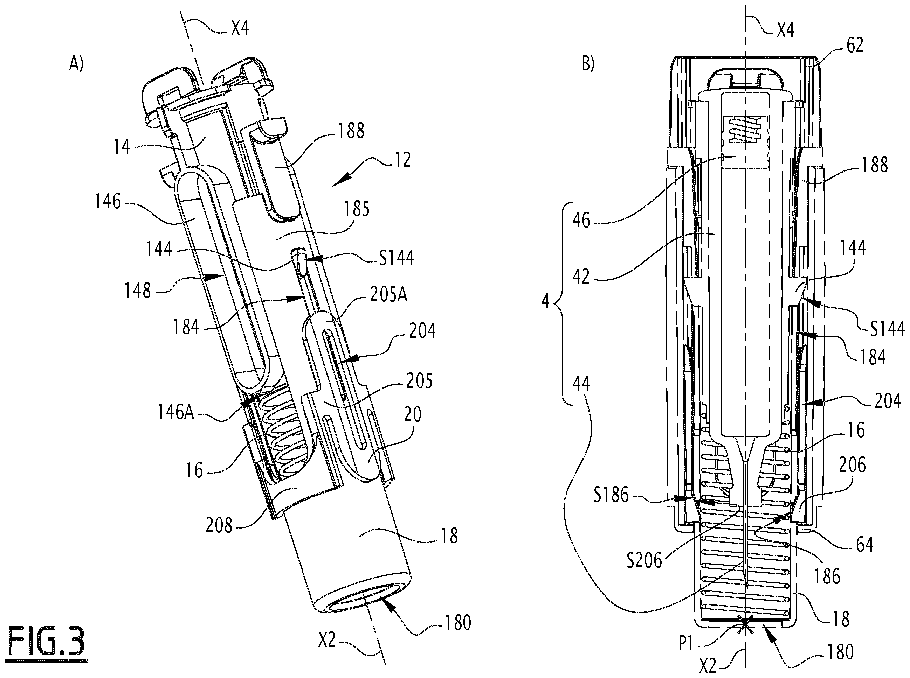

In assembled configuration of the safety system 12, the needle cover 18 is mounted around the sleeve 14, with the ribs 146 engaged into the notches 189 and the key elements 144 engaged into the slots 184, as visible on figures 3 to 5. In this configuration, the elongated portions 185 are located around the sleeve 14, in particular its surface S142.

Advantageously, in this assembled configuration, the longitudinal axes X14 and X18 coincide. In this assembled configuration, the needle cover 18 protrudes axially out of the sleeve 14 through an end opening 140 defined by the sleeve 14 and which is oriented towards the second end 64 of the body 6 when the safety system 12 is integrated within the injection device 2.

146A denotes an extremity of a rib 146 close to the end opening 140.

When the safety system 12 is incorporated as a part of the injection device, the longitudinal axes X14 and X18 are aligned on the longitudinal axis X2.

Due to the relative longitudinal dimensions of the key elements 144 and of the slots 184, namely to the fact that the slots 184 are significantly longer than the key elements 144, and to the fact that the notches 189 are opened in the direction of the lugs 188, the needle cover 18 can translate along the longitudinal axis X2, with respect to the sleeve 14.

The safety system 12 also includes a locker 20, which is in a form an annular element centered on a longitudinal axis X20. In assembled configuration of the safety system 12, the locker 20 is mounted around the needle cover 18. Advantageously, in this assembled configuration, the axes X14, X18 and X20 coincide.

The locker 20 includes a body 202, preferably made of a polymer which can be the same or different than the materials of the sleeve 14 and the needle cover 18.

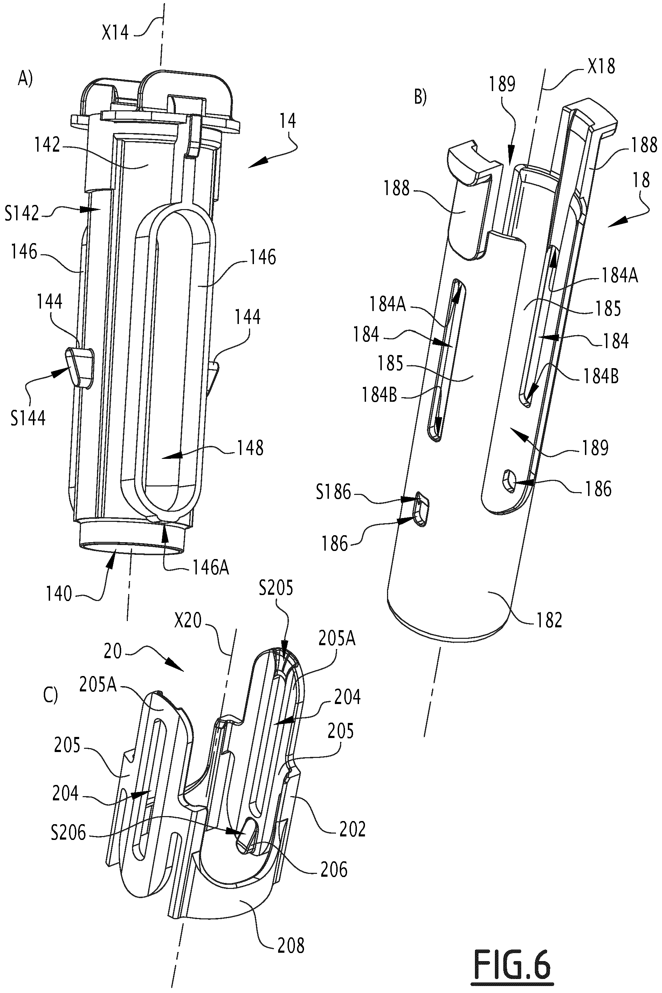

The body 202 defines two longitudinal apertures 204 parallel to the longitudinal axis X20 and which are located diametrically oppositely with respect to the longitudinal axis X20, and two heels 206, which project radially from the body 202 towards the longitudinal axis X20. One of the heels is visible on figure 6 and the two heels are visible on insert B of figures 3, 4 and 5. The two heels 206 are located diametrically oppositely with respect to the longitudinal axis X20 and are internal with respect to the body 202, as they are located within the volume defined by this body.

In a non-represented variant of the invention, the locker 20 includes one aperture 204 and/or one heel 206 only.

A surface S206 of each heel 206 facing the opposite heel is inclined with respect to the longitudinal axis X20 in such a way that the radial thickness of each heel 206 decreases along the longitudinal axis X20 in the direction of the adjacent longitudinal aperture 204. In this way the surfaces S206 are formed as ramp surfaces.

The longitudinal apertures 204 are each formed in an elongated tab 205 of the locker 20. Two branches 208 connect further the two elongated parts 205. The tabs 205 and the branches 208 belong to the body 202.

The tabs 205 are preferably elastically deformable.

Advantageously, the heels 206 are provided on the tabs 205, preferably on their inner side.

Advantageously, each one of parts 14, 18 and 20 is made in one piece, in other words monobloc.

In mounted configuration of the safety system 12, the locker 20 is mounted around the needle cover 18 and partly around the sleeve 14.

180 denotes an end opening of the needle cover 18 which is also oriented towards the second end 64 of the body 6 when the safety system 2 is integrated within the injection device 2. During injection of the content of the barrel 42, for example into a human or animal body, the needle 44 protrudes out of the needle cover 18 through the opening 180.

During use of the injection device 2, the needle cover 18 can take various positions along the longitudinal axis X2, with respect to the sleeve 14, several of them being represented respectively on figures 3, 4 and 5.

Once the puller 8 along with the cap 10 and the needle cap 48 have been removed, the safety system 12 is in the first configuration of figure 3, where the geometrical center point of the end opening 180 coincides with a point P1 of the longitudinal axis X2. In other words, the point P1 corresponds to a first position of the needle cover 18 with respect to the sleeve 14, along the longitudinal axis X2. This position is represented on figure 3.

In this position, each heel 206 is engaged within a recess 186, which couples the locker 20 with the needle cover 18 and the surfaces S206 of the heels rest on the ramp surfaces S186 of the recesses 186. As a result, the needle cover 18 is coupled with the locker 20 in translation when the needle covers translates in the direction D2. However, when the needle cover 18 is translated in the direction D2', a relative sliding movement is possible between the surfaces S186 and S206 and the needle cover 18 can be translated in the direction D2' with respect to the locker 20. On the other hand, the key elements 144 of the sleeve 14 are engaged within the longitudinal slots 184 of the needle cover 18. The spring 16 may urge the needle cover 18 away from the sleeve 14, in the direction D2'. The movement of the needle cover 18 in the direction D2' is limited by the key elements 144 coming into abutment with first extremities 184A of the longitudinal slots 184 opposite to the opening 180.

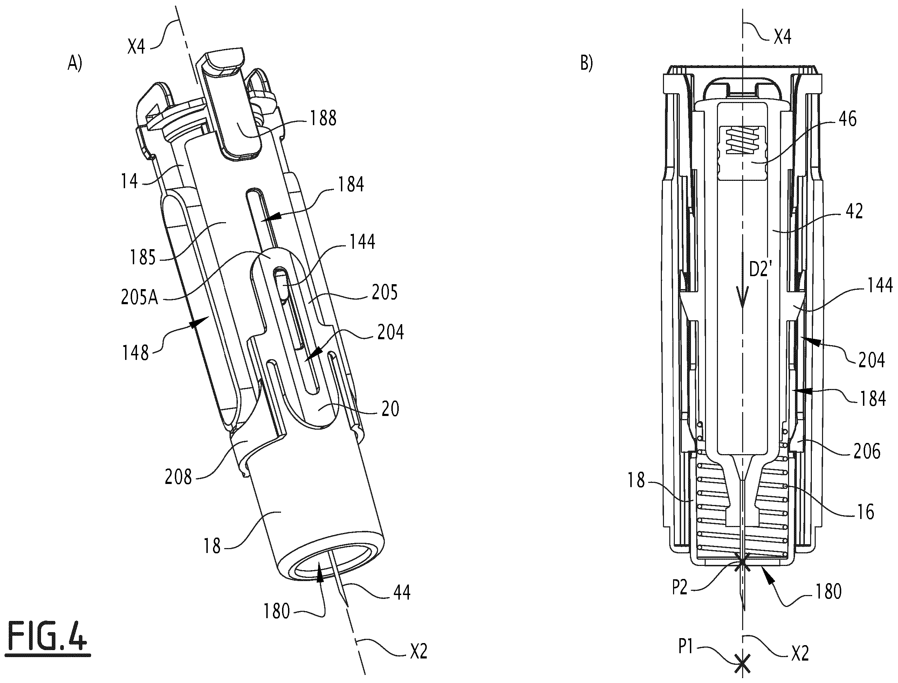

From the first configuration of figure 3, if the needle cover 18 rests on a surface, such as, for example, the skin of a human or animal body, it is possible to push down the body 6 along the longitudinal axis X2, in the second direction D2', which moves the needle cover 18 with respect to the sleeve 14, from the configuration of figure 3 to the configuration of figure 4, where the center point of the opening 180 coincides with a second point P2 of the longitudinal axis X2. This defines a second position of the needle cover 18 with respect to the sleeve 14 along the longitudinal axis X2. This position is represented on figure 4.

The needle cover 18 is able to translate, in particular configured to translate, with respect to the sleeve 14 between the first position and the second position. The axial translational movement of the needle cover 18, with respect to the sleeve 14 and along the longitudinal axis X2, from its first position represented on figure 3, to its second position represented on figure 4, occurs along the first direction D2, against the elastic biasing force exerted by the spring 16.

During its axial translational movement, between its first position and its second position, the needle cover 18 carries the locker 20 because the locker is coupled to the needle cover 18 by the engagement of the heels 206 into the recesses 186, as it is described above. This movement occurs up to when each one of two branches 208 of the locker 200 comes into abutment against an extremity 146A of a rib 146. This abutment defines the second position of the needle cover 18 corresponding to the full retraction of the needle cover 18 when the needle is inserted into, for example, a human or animal skin.

During the axial translational movement of the needle cover 18 between its first position and its second position, the key elements 144 of the sleeve are engaged in the longitudinal slots and travel along the longitudinal slots 184, from a position close to their first extremities 184A to an intermediate position.

At the end of the movement of the locker 20 driven by the needle cover 18 going from the first position to the second position, when the needle cover 18 reaches its second position, the tabs 205 of the body 202 reach the key elements 144 and deforms elastically, at the level of a tip 205A opposite to the branches 208, so that each key element 144 engages within a longitudinal aperture 204.

This occurs, preferably, by elastic deformation of the locker 20, at the level of the tabs 205.

In a non-represented variant of the invention each key element 144 is flexible and engages within a longitudinal aperture by elastic deformation of the key element and/or the locker 20.

This is further facilitated by the fact that the key elements 144 have a wedged shape with a surface S144 inclined with respect to the longitudinal axis X14 and converging towards the end opening 140. This is also facilitated by the fact that the tips 205A each have an internal surface S205, which is diverging in a direction going away from the heels 206 and the branches 208.

In other words, the surfaces S144 and S205 form ramps, which facilitate the introduction of the key elements 144 into the longitudinal apertures 204. Due to this introduction of the key elements 144 into the longitudinal apertures 204 and to the abutment of the branches against the extremities 146A, the locker 20 couples with the sleeve 14 and becomes fixed with this sleeve.

As there is no further relative axial movement between the locker 20 and the key 14, once the key elements 144 are engaged in the longitudinal apertures 204, they remain in these apertures.

Thus, the locker 20 is configured to be selectively coupled to the needle cover 18 and to the sleeve 14.

From the second position of figure 4, once injection is terminated and the needle cover 18 does not rest on the skin of the human or animal body any more, the needle cover 18 is pushed back by the spring 16 into a third position where the geometrical center point of the end opening 180 is aligned with a third point P3 of the longitudinal axis X2.

This defines a third position of the needle cover 18, along the longitudinal axis X2.

Here, the points P1 and P3 coincide. In other words, the first and third positions of the needle cover 18, along the longitudinal axis X2, are the same.

The needle cover 18 is able to translate, in particular configured to translate, with respect to the sleeve 14, between the second position and the third position.

The second translational movement of the needle cover 18, from its second position to its third position, occurs in the second direction D2'. It is a return axial movement, opposite to the first translational movement of the needle cover 18, in the first direction D2. Starting from the second position of figure 4 where the locker 20 is hooked onto the sleeve 40 by engagement of the key elements 144 into the longitudinal apertures 204, it is not possible for the locker 20 to follow the return axial displacement of the needle cover 18 towards the third position.

At the beginning of this return axial movement, the needle cover 18 and the locker 20 decouple due to the fact that the sliding movement between the surfaces S206 and S186 causes that the heels 206 move out from the recess 186 by elastic deformation of the locker 200. This extraction is facilitated by fact that the tabs 205 are elastically deformable.

Thus, when the needle cover 18 axially translates, from its second position to its third position, in the second direction D2', it translates with respect to the sleeve 14 and also with respect to the locker 20 since the locker 20 remains stationary with the sleeve 14.

During the axial translational movement of the needle cover 18 between its second position and its third position, the key elements 144 remain engaged in the longitudinal slots and travel back along the longitudinal slots 184, from their intermediate position to their position close to their first extremities 184A.

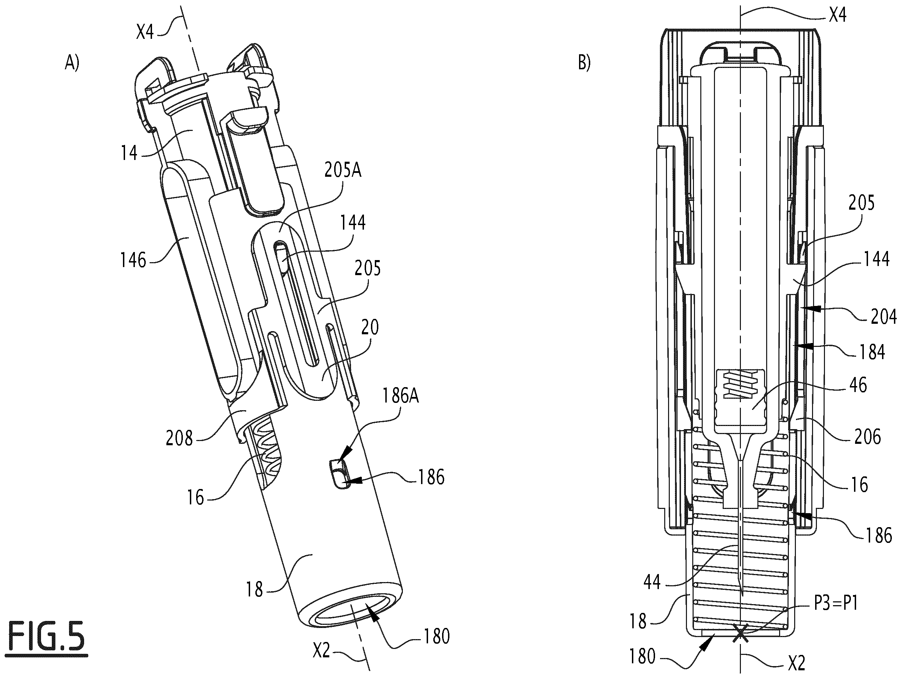

When it reaches its third position represented on figure 5, the needle cover 18 is locked in this position due to the fact that the heels 206 elastically enter, and engage within, the longitudinal slots 184, at the level of their second extremities 184B opposite to their first extremities 184A. This engagement of the heels 206 into the longitudinal slots 184 occurs via elastic deformation of the tabs 205.

The cooperation of the heels 206 with the second extremities 184B of the longitudinal slots 184 prevents a further movement of the needle cover 18 in the first direction D2. Thus, the needle cover 18 is securely locked in its third position, which is the same as its first position, where it protects the needle 44 from the exterior and prevents needle-stick injuries.

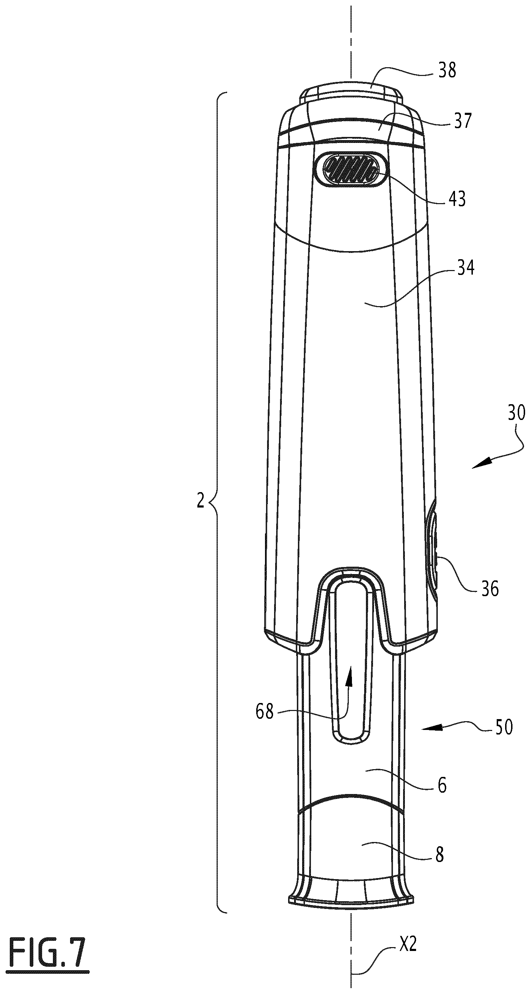

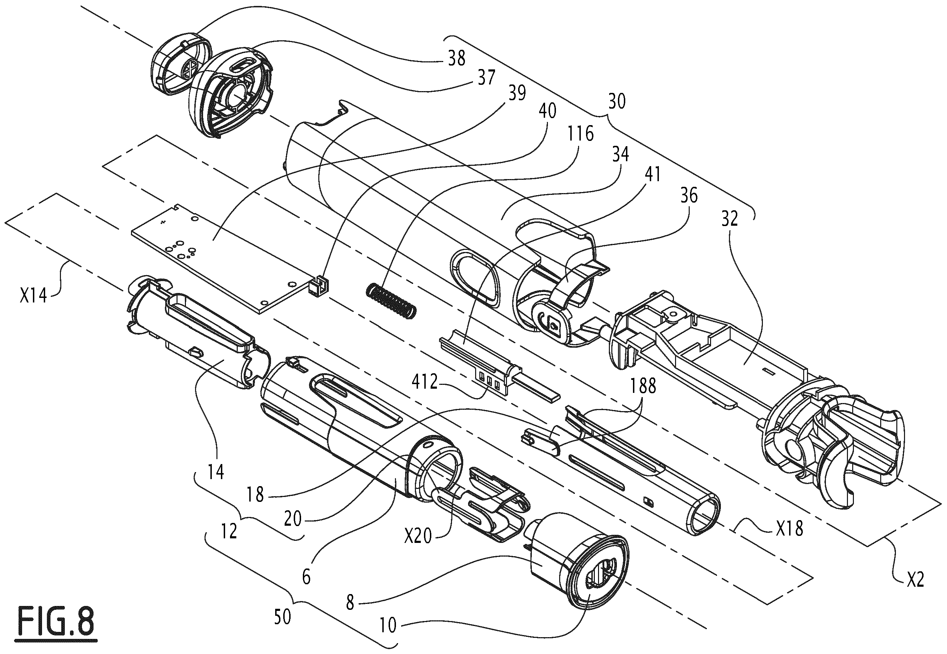

In the second embodiment of the invention represented on figures 7 and 8, the same elements as in the first embodiment have the same references. If a reference is mentioned in the following description without being shown on figures 7 and 8 or shown on these figures without being mentioned in the description, it designates the same element as the one bearing the same reference in the first embodiment. Unless otherwise specified, the second embodiment works as the first embodiment. Hereafter, one describes mainly the differences between the first and second embodiments.

In the second embodiment of the invention, the injection device 2 includes a reusable module 30 couplable with a disposable module 50. The reusable module 30 comprises a frame 32, a shell 34, a locking button 36, a light indicator 37, a push button 38, a printed circuit board 39 and a non-represented motor configured for pushing the piston of a non-represented syringe, similar to the syringe 4 represented on figures 2 to 5. A slider control button 43, represented on figure 1 only and mounted on the shell 34, allows controlling the speed of displacement of the piston of a syringe, when it is moved in the barrel 42 by the non-represented motor.

The reusable module 30 also includes an optical sensor 40 and a shuttle 41, mobile along a direction parallel to the longitudinal axis X2 of the injection device 2. This shuttle is in contact with one of the lugs 188 of the needle cover 18. A spring 116 exerts a force on the shuttle 41 towards the needle cover 18 providing more stable contact between these elements. Thus, the shuttle 41 is a pushing element. The shuttle 41 is equipped with a barrier 412 configured to move in front of the light detector of the optical sensor 40, which allows detecting a position of the needle cover 18 along the longitudinal axis X2.

Once the disposable module 50 is connected to the reusable module 50, the shuttle 41 is in abutment against the lugs 188 of the needle cover 18, under the action of the spring 116. Thus, the shuttle 41 and the needle cover 18 are coupled in translation, along the longitudinal axis X2 and with reference to the frame 32.

The injection device 2 of the second embodiment also includes a body 6, a puller 8 and a cap 10 similar to the ones of the first embodiment and a safety system 12 which includes a sleeve 14, a needle cover 18 and a locker 20. These parts 14, 18 and 20 are similar to the ones of the first embodiment, but for the shape of the lugs 188 of the needle cover 18, which are configured for interacting with the shuttle 41. Apart from its interaction with the shuttle 41, the safety system 12 of the second embodiment works substantially in the same way as the safety system 12 of the first embodiment.

In this second embodiment, parts 6, 8, 10, the safety system 12 and the non-represented syringe together belong to a disposable module 50 of the injection device 2, which is discarded when the content of the needle has been injected.

A difference with the first embodiment is that the spring 116, which biases the needle cover toward its first and third positions, belongs to the reusable module 30. Hence, the disposable module 50 may be composed of only plastic parts, which makes the disposable module cheaper and more recyclable.

In all embodiments of the invention, the safety system 12 improves the safety of the operation of the injection device 2. When moving from its first position to its second position, the needle cover 18 carries the locker 20 up to a position where the locker couples with the sleeve 14 and where it remains when the needle cover 18 goes into its third position, which is the same as its first position. The needle cover 18 is blocked by the locker 20 in this third position, which prevents access to the needle of the syringe after use of the device. At the same time, the needle is hidden before each use steps of the injection device which reduces the stress, in particular for the people suffering from needlephobia.

The embodiments and variants of the invention mentioned here above may be combined, in any technically feasible way, in order to generate new embodiments of the invention, in the framework of the appended set of claims.

## Claims

1. A safety system (12) for an injection device (2) comprising a body (6),
the safety system comprising
- a sleeve (14) fixed in the body (6);
- a needle cover (18) able to translate with respect to the sleeve (14) between a first position, a second position and a third second position in two opposite longitudinal directions (D2, D2');
- a locker (20) able to translate in a longitudinal direction (D2) and configured to be selectively coupled to the needle cover (18) and to the sleeve (14),
wherein
- the needle cover (18) is configured to translate, in a first longitudinal direction (D2), from the first position to the second position, along with the locker (20);
- the needle cover (18) is configured to translate with respect to the locker (20), in a second longitudinal direction (D2'), opposite to the first longitudinal direction, from the second position to the third position;
- the third position is the same as the first position; and
- after moving from the second position to the third position, the needle cover (18) is locked in the third position.

2. The safety system of claim 1, wherein the locker (20) is ring shaped and mounted around the needle cover (18) in assembled configuration of the safety system (12).

3. The safety system of any preceding claim, wherein, when it reaches the second position, the locker (20) couples with the sleeve (14) and becomes fixed with the sleeve.

4. The safety system of any preceding claim, further comprising a biasing element (16; 116) for biasing the needle cover (18) toward its first position.

5. The safety system of any preceding claim, wherein
- the needle cover (18) comprises at least one longitudinal slot (184);
- the sleeve (14) comprises at least one key element (144) protruding radially with respect to a peripheral surface (S142) of the sleeve;
- each key element (144) is engaged in one longitudinal slot (184) of the needle cover and travels along this longitudinal slot when the needle cover moves from the first position to the second position and from the second position to the third position.

6. The safety system of claim 5, wherein
- the needle cover (18) includes two elongated portions (185) disposed around the sleeve;
- each elongated portion (205) is equipped with a longitudinal slot (184);
- the sleeve comprises two key elements (144), preferably diametrically opposed; and
- each key element protrudes outwardly from the outer peripheral surface (S142) and is engaged in a respective longitudinal slot (184).

7. The safety system of any preceding claim, wherein
- the locker (20) includes at least one aperture (204);
- the sleeve comprises at least one key element (144) protruding radially with respect to a peripheral surface (S142) of the sleeve;
- each key element (144) engages into one aperture (204) of the locker when the needle cover (18) reaches its second position and remains in this aperture when the needle cover moves from the second position to the third position.

8. The safety system of claim 7, wherein engagement of the key element (144) into the aperture (204) occurs by elastic deformation of the locker (20).

9. The safety system of any preceding claim, wherein the locker (20) comprises at least one locking heel (206) engaged in a corresponding recess (186) of the needle cover (18), when the needle cover (18) is in its first position before moving to its second position and when the needle cover moves from its first position to its second position, and wherein the heel (206) enters the longitudinal slot (184) of the needle cover when the needle cover reaches its second position and stays in the longitudinal slot when the needle cover moves from its second position to its third position.

10. The safety system of claim 9, wherein the heel (206) is provided on a deformable tab (205) of the locker and extends radially with respect to this deformable tab.

11. An injection device (2) comprising a syringe (4), with at least a barrel (42) and a needle (44), and a safety system (12) according to any preceding claim for shielding the needle (44) from the exterior.

12. The injection device of claim 11, comprising a reusable module (30) and a disposable module (50), which includes the safety system (12).

13. The injection device of claim 12, wherein the reusable module (30) comprises a frame (32) and a pushing element (41), wherein after connecting the reusable module (30) with the disposable module (50), the pushing element (41) is coupled with the needle cover (18) in translation with reference to the frame (32).

14. The injection device of claim 13, wherein the biasing element (116) is part of the reusable module (30) and located between the pushing element (41) and the frame (32).

15. The injection device of any of claims 12 to 14, wherein the injection device comprises a motor for providing a force for the injection.
